# EUROPÄISCHE PATENTANMELDUNG

(11) **EP 2 390 506 A1**
(43) Veröffentlichungstag der Anmeldung: **30.11.2011**
(21) Anmeldenummer: 11159426.3
(22) Anmeldetag: 23.03.2011
(51) Int. Cl.: F04B 49/06, F04B 53/16, A61M 5/142, A61M 5/145, A61M 5/168, B60T 8/36, F15B 1/26, E03B 11/16

(54) **Vorrichtung zur Fluidversorgung und Steuerung eines Fluidiksystems**

(30) Priorität: 23.03.2010 DE 102010016106
(71) Anmelder: Andreas Hettich GmbH&Co. KG, 78532 Tuttlingen (DE)
(72) Erfinder: Gehring, Frank K., 72364, Obernheim (DE)
(74) Vertreter: Borchert, Uwe Rudolf

(57) **Zusammenfassung**

Die Erfindung betrifft eine Vorrichtung (10) zur Fluidversorgung und Steuerung eines Fluidiksystems, umfassend ein Gehäuse (12), eine in das Gehäuse (12) integrierte Steuerungseinheit, sowie zumindest eine in das Gehäuse (12) integrierte Pumpe und zumindest ein in das Gehäuse (12) integriertes Ventil (22), die mit der Steuerungseinheit signaltechnisch verbunden sind und durch die Steuerungseinheit steuerbar sind, wobei das Gehäuse (12) eine, von außen zugängliche, standardisierte fluidische Anschlussstellen (14, 16, 20) aufweisende Anschlussplatte (24, 26) zum Anschluss externer fluidischer Anschlüsse aufweist, und die zumindest eine Pumpe und das zumindest eine Ventil (22) im Inneren des Gehäuses (12) mit den Anschlussstellen (14, 16, 20) der Anschlussplatte (24, 26) verbunden sind.

## Beschreibung

Die Erfindung betrifft eine Vorrichtung zur Fluidversorgung und Steuerung eines fluidiksystems.

Vorrangig bei fluidischen Versuchsaufbauten ist immer die mehr oder minder variable Verwendung von Pumpen und Ventilen. Für jede Versuchsanordnung muss nach dem Stand der Technik immer eine eigene Steuerungssoftware programmiert werden. Die Ventile und Pumpen werden in der Regel über serielle Schnittstellen angesteuert. Entsprechend müssen die Treiber auch in die speziell programmierte Steuerungssoftware eingebunden werden. Dies ist nicht immer einfach und bedarf ausreichende Sachkenntnis.

Der Erfindung liegt die Aufgabe zu Grunde eine Vorrichtung zur Versorgung und Steuerung eines Fluidiksystems anzugeben, welches ermöglicht über einfache Eingabe ohne spezielle Kenntnisse ein Fluidiksystem zu versorgen und zu steuern.

Diese Aufgabe wird durch die Merkmale des Anspruches 1 gelöst.

Die erfindungsgemäße Vorrichtung ist mit zumindest einer Pumpe, zumindest einem Ventil und zumindest einer Steuereinheit versehen, die in einem gemeinsamen Gehäuse untergebracht sind. Die Pumpen und Ventile können über die Steuereinheit auf einfache Weise angesprochen werden.

Insbesondere ist die Steuereinheit über ein Touchpanel einstellbar, welches ebenfalls in das Gehäuse integriert ist. Vorzugsweise kann das Touchpanel als Touchscreen ausgebildet sein. Ferner können alternativ auch andere Eingabemittel vorgesehen sein.

Die Anschlussstellen der Pumpe und Ventile sind vorzugsweise leckagefrei an die Anschlussstellen des Gehäuses angeschlossen. Die Anschlussstellen können somit extern an ein Fluidiksystem angeschlossen werden. Die Ventile und Pumpen sind zudem mit der Steuereinheit verbunden.

Der Anwender ist durch diese Vorrichtung in der Lage schnell und einfach ein funktionierendes Fluidiksystem erzeugen zu können, indem einfach die entsprechenden Ventile über die Anschlussplatte bedarfsgerecht extern fluidisch verbunden werden.

In einer besonders vorteilhaften Ausgestaltung können die Ventile als Mehr-Wege-Ventile, beispielsweise Selektor oder Verteilerventile ausgebildet sein.

Pumpengeschwindigkeit und Ventilstellungen können über Steuerungslisten an das Steuersystem übermittelt werden. Der Anwender muss sich nicht mehr um die Kommunikation zwischen Steuersystem und Pumpen und Ventile kümmern. Diese ist bereits vorimplementiert und im Steuersystem festgelegt. Dennoch sind Eingabemöglichkeiten vorgesehen auch direkt auf die Gerätetreiber zuzugreifen.

Vorzugsweise können wenigstens die Ventile derart angeordnet sein, dass diese über eine Öffnung des Gehäuses zugänglich sind, um die fluidische Verschaltung anpassen zu können.

In einer besonders vorteilhaften Ausführungsform sind die Komponenten in eine Klappe angeordnet. Durch diese sind Pumpen und Ventile zugänglich, was ein Ändern der standardisierten Anschlüsse ermöglicht und die Anpassung auf Spezialfälle ermöglicht. Dieses ist beispielsweise von Bedeutung, falls kurze fluidische Wege notwendig sind, oder eine Anpassung der Pumpen in ihren Förderbereichen notwendig ist. In der Regel können Pumpen, die unterschiedliche Fördermengen aufweisen fluidisch ausgetauscht werden, ohne diese auch elektrisch bzw. steuerungstechnisch anpassen zu müssen. Eine entsprechende Zugänglichkeit erhöht deutlich die Flexibilität und den Anwendungsbereich der Vorrichtung.

Ein weiterer Vorteil der Erfindung ergibt sich aus der Möglichkeit die Fluidsteuerung mit anderen Systemen zu synchronisieren. Dafür ist insbesondere eine Schnittstelle für ein TTL-Signal vorgesehen. Das TTL Signal kann sowohl empfangen als auch gesendet werden. Das TTL Signal triggert und stoppt den Prozessablauf. Dies ist vor allem für angeschlossenen Messsysteme von großer Bedeutung.

Neben dem TTL Signal kann die Vorrichtung weitere Schnittstellen aufweisen. Herkömmlicher Weise sind dies LAN, USB, Firewire, RS 488, RS 232, Bildschirm etc. Über diese Schnittstellen können beispielsweise die von der Steuereinheit erzeugten Protokolle abgelegt oder ausgedruckt werden. Zudem können über diese Schnittstellen die Steuerungslisten eingegeben werden.

Die Steuerungslisten können im Excel-Format vorliegen oder einfach nur tab-stop-getrennte .txt-Dateien, oder ähnliche gängige digitale Listenformate sein.

In einer weiteren vorteilhaften Ausführungsform, sind zwei Pumpen und zwei Ventile vorgesehen. Dies ermöglicht das Vermischen von Fluiden. Die Ventile weisen je einen Eingangspfad auf, der abhängig von der Schaltstellung mit 4 Ausgangspfaden verbunden werden kann. Die Schaltstellungen können über Steuerungslisten zeitabhängig eingestellt werden.

Durch die standardisierten Anschlüsse an der Anschlussplatte können beliebige Fluidiksysteme angeschlossen werden. Dies gilt insbesondere für mikrofluidische Plattformen, welche dadurch einfach mit einer fluidischen Versorgungs- und Steuereinheit verbunden werden können.

Weitere Vorteile, Merkmale und Anwendungsmöglichkeiten der vorliegenden Erfindung ergeben sich aus der nachfolgenden Beschreibung in Verbindung mit den in der Zeichnung dargestellten Ausführungsbeispiels.

Die Erfindung wird im Folgenden anhand des in der Zeichnung dargestellten Ausführungsbeispiels näher beschrieben. In der Beschreibung, in den Ansprüchen und in der Zeichnung werden die in der unten aufgeführten Liste der Bezugszeichen verwendeten Begriffe zugeordneten Bezugszeichen verwendet.

In der Zeichnung bedeutet:
- Fig. 1: eine perspektivische Ansicht einer Fluidsteuervorrichtung;
- Fig. 2: eine perspektivische Ansicht eines Fluidversorgungssystems mit geöffneter Revisionsklappe;

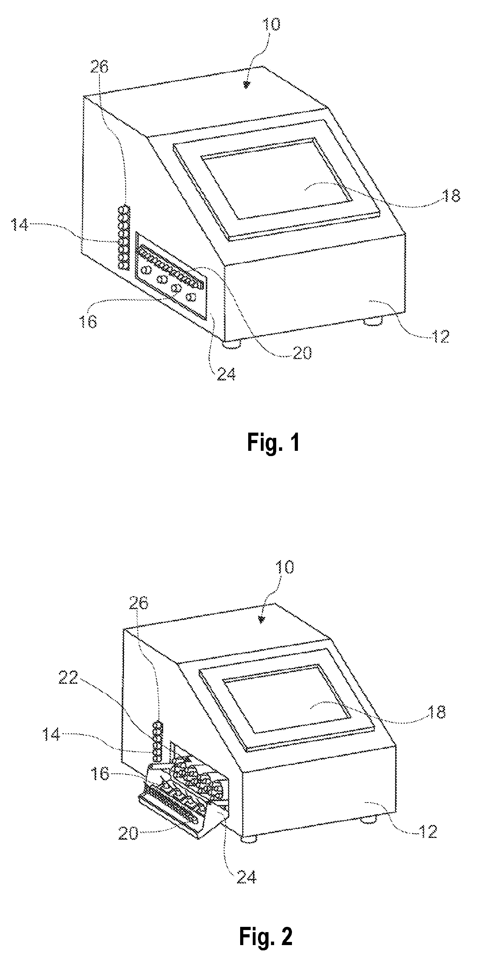

Fig. 1 zeigt die perspektivische Ansicht eines Fluidversorgungssystems 10 mit einem Gehäuse 12. Innerhalb des Gehäuses 12 sind vier Pumpen angeordnet, die jeweils mit ihren Ein- und Ausgängen mit standardisierten Anschlussstellen leckagefrei fluidisch verbunden sind. Über diese Pumpenanschlussstellen 14 können beispielsweise Vorratsbehälter, fluidische Schaltungen oder mit den in das Gehäuse integrierte Ventile verbunden werden. Die Ventile innerhalb des Gehäuses sind ebenfalls mit Ventilanschlussstellen 16 am Gehäuse verbunden. Die Anschlussstellen sind ebenfalls standardisiert ausgeführt. Die Ventile weisen in dieser Ausführungsform jeweils einen Eingang und vier Ausgänge auf. Die Ventile können somit über standardisierte Fluidikanschlüsse extern kontaktiert werden. So kann auf einfache Weise ein mikrofluidisches System verschalten werden.

Die Pumpenanschlüsse 14 sind in einer Anschlussplatte 26 zusammen aufgenommen, die ein Teil der Gerätegehäusewand darstellt. Die Ventilanschlüsse 16, 20, sind hingegen in einer separaten Revisionsklappe 24 angeordnet. Die Ventile 22 können sowohl als Selektor- als auch als Verteilerventile betrieben werden. Im Falle eines Selektorbetriebs stellen die Anschlüsse 16, die Eingänge und die vier jeweils zugeordneten Anschlüsse 20 die Ausgänge des Ventils dar. Im anderen Fall ist die Zuordnung umgekehrt.

Darüber hinaus ist im Gehäuse 12 eine Recheneinheit integriert. Die Pumpen und Ventile sind über serielle Schnittstellen mit dieser Recheneinheit verbunden. Diese Recheneinheit übernimmt die Steuerung des Fluidversorgungssystems 10. Um Prozessabläufe auf die Steuereinrichtung 24 übertragen, können einfache Listen an die Steuereinheit übermittelt werden. Die Übermittlung der Eingabelisten kann über vorgesehene Schnittstellen erfolgen. Entsprechende Schnittstellen sind LAN, USB und Firewire.

Um eine weitere Mensch- Maschine Schnittstelle bereitzustellen, ist im Gehäuse 12 ferner ein Touchpanel 18 integriert. Über dieses Touchpanel 18 können Konfigurationseinstellungen vorgenommen werden oder Prozessablauflisten generiert werden.

Diese Vorrichtung hat den Vorteil, dass beliebige Fluidiksysteme über standardisierte Anschlüsse betrieben und versorgt werden können. Der Anwender muss sich dabei nicht mehr um die Integration der Schnittstellentreiber kümmern, sondern kann einfach, beispielsweise über Excellisten einen Prozessablauf vorgeben. Zudem kann das Fluidiksystem über ein TTL-Signal mit anderen Systemen, insbesondere Messsysteme synchronisiert werden.

Fig. 2 zeigt die Fluidikversorgungsvorrichtung mit geöffneter Revisionsklappe 20 in perspektivischer Ansicht. Die Anschlussplatte, an welcher die fluidischen Anschlüsse 16, 24 angeordnet sind, ist als Revisionsklappe 20 ausgebildet. Die Revisionsklappe 20 ermöglicht den Zugang zu den Ventilen 22, sodass ein Anwender spezielle Konfigurationseinstellungen vornehmen kann. Beispielsweise kann dies eine Rolle spielen, wenn besonders kurzer Fluidikwege notwendig sind. Zudem ermöglicht es den Austausch bzw. die Wartung der Ventile 22.

Durch diese Vorrichtung wird es einem Anwender ermöglicht, ohne Programmierkenntnisse ein Fluidiksystem mit der vorgegebenen Verschaltungsmöglichkeit einfach und schnell herzustellen.

### Bezugszeichenliste

- 10: Fluidversorgungssystem
- 12: Gehäuse
- 14: Pumpenanschlüsse
- 16: Ventileingang
- 18: Touchpanel
- 20: Ventilausgang
- 22: Ventile
- 24: Revisionsklappe
- 26: Anschlussplatte

## Patentansprüche

1. Vorrichtung (10) zur Fluidversorgung und Steuerung eines Fluidiksystems, umfassend ein Gehäuse (12), eine in das Gehäuse (12) integrierte Steuerungseinheit, sowie zumindest eine in das Gehäuse (12) integrierte Pumpe und zumindest ein in das Gehäuse (12) integriertes Ventil (22), die mit der Steuerungseinheit signaltechnisch verbunden sind und durch die Steuerungseinheit steuerbar sind, wobei das Gehäuse (12) eine, von außen zugängliche, standardisierte fluidische Anschlussstellen (14, 16, 20) aufweisende Anschlussplatte (24, 26) zum Anschluss externer fluidischer Anschlüsse aufweist, und die zumindest eine Pumpe und das zumindest eine Ventil (22) im Inneren des Gehäuses (12) mit den Anschlussstellen (14, 16, 20) der Anschlussplatte (24, 26) verbunden sind.

2. Vorrichtung nach Anspruch 1, **dadurch gekennzeichnet, dass** in dem Gehäuse (12) ein Touchpanel (18) integriert ist, mittels dem die Eingabe der Steuerung vorgesehen ist.

3. Vorrichtung nach einem der vorangehenden Ansprüche, **dadurch gekennzeichnet, dass** wenigstens die Ventile (22) zugänglich durch eine Öffnung des Gehäuses (24) montiert sind.

4. Vorrichtung nach einem der vorangehenden Ansprüche, **dadurch gekennzeichnet, dass** die Prozessabfolge mittels Eingabelisten festlegbar ist.

5. Vorrichtung nach einem der vorangehenden Ansprüche, **dadurch gekennzeichnet, dass** eine Schnittstelle für ein TTL-Signal vorgesehen ist, die eine Synchronisation mit anderen Geräten ermöglicht.

6. Vorrichtung nach einem der vorangegangenen Ansprüche, **dadurch gekennzeichnet, dass** weitere Schnittstellen, wie CAN, Ethernet, oder USB in die Vorrichtung integriert sind, über die eine Interfunktionalität mit anderen Komponenten ermöglicht ist.
